# EUROPÄISCHE PATENTSCHRIFT

(11) **EP 2 051 719 B1**
(45) Veröffentlichungstag und Bekanntmachung des Hinweises auf die Patenterteilung: **03.09.2014**
(21) Anmeldenummer: 07786404.9
(22) Anmeldetag: 28.07.2007
(51) Int. Cl.: A61K 35/56, A61K 38/39, A61P 17/02

(54) **ZUBEREITUNG MIT MARINEM KOLLAGEN ZUR PROTEINASEHEMMUNG**
PREPARATION WITH MARINE COLLAGENS FOR PROTEASE INHIBITION
PRÉPARATION À BASE DE COLLAGÈNE MARIN POUR INHIBER LA PROTÉINASE

(30) Priorität: 16.08.2006 DE 102006038252
(43) Veröffentlichungstag der Anmeldung: 29.04.2009
(73) Patentinhaber: Lohmann & Rauscher GmbH & Co. KG, 56567 Neuwied (DE)
(72) Erfinder: ALUPEI, Corneliu, Lulian, 56564 Neuwied (DE); RUTH, Peter, 56581 Melsbach (DE); WILHELMS, Tim, Axel, 56068 Koblenz (DE); ROHRER, Christian, 4020 Linz (AT)
(74) Vertreter: Flaccus, Rolf-Dieter
(86) Internationale Anmeldenummer: PCT/EP2007/006694
(87) Internationale Veröffentlichungsnummer: WO 2008/019756

(56) Entgegenhaltungen:
- WO-A-02/102831
- WO-A-2006/034568
- WO-A-2006/089660
- DE-A1- 10 010 113
- US-A- 5 819 748
- DATABASE WPI Week 199948 Derwent Publications Ltd., London, GB; AN 1999-564719 XP002454959 & JP 11 244324 A (IHARA SUISAN KK) 14. September 1999 (1999-09-14)
- SWATSCHEK D ET AL: "Microparticles derived from marine sponge collagen (SCMPs): preparation, characterization and suitability for dermal delivery of all-trans retinol" September 2002 (2002-09), EUROPEAN JOURNAL OF PHARMACEUTICS AND BIOPHARMACEUTICS, ELSEVIER SCIENCE PUBLISHERS B.V., AMSTERDAM, NL, PAGE(S) 125-133 , XP004377354 ISSN: 0939-6411 das ganze Dokument

## Beschreibung

Die vorliegende Erfindung betrifft die Verwendung von aus marinen Schwämmen gewonnenem Kollagen zur Herstellung von pharmazeutischen Zubereitungen zur Behandlung von chronischen Wunden.

Die Matrix-Metallo-Proteasen (Matrix-Metallo-Proteinasen / Matrix-Metallo-Peptidasen / MMP) bilden eine Enzym-Familie, die in verschiedenen Zellen gebildet wird und die für die extrazelluläre Degradation von Makromolekülen verantwortlich ist. Der Name "Matrix-Metallo-Proteasen" ist darauf zurückzuführen, daß die katalytische Aktivität der Proteasen auf einem Zinkatom im katalytischen Zentrum beruht und die strukturelle Stabilität des Proteins durch Kalium gewährleistet wird.

MMPs werden in vier verschiedene Klassen eingeteilt, wobei diese Einteilung unter anderem aufgrund der Domänenstruktur und bevorzugter Substrate erfolgt, so daß beispielsweise Kollagenasen und Gelatinasen unterschieden werden.

Aufgabe der MMPs ist die Hydrolyse von Komponenten der extrazellulären Matrix wie Kollagenen, Gelatine, Nektinen, Lamininen, Elastin und Core-Proteinen der Proteoglykane. Sie spielen daher bei den physiologischen Prozessen der Gewebeumwandlung, beispielsweise in der Embryonalentwicklung, dem Wachstum, aber auch in der Wundheilung und in pathobiologischen Prozessen eine entscheidende Rolle.

Während MMPs unter "normalen Bedingungen" in Haut, Gewebe und Gelenken praktisch nicht oder nur in geringen Konzentrationen nachgewiesen werden können, treten sie bei Verletzungen der Haut und bei Erkrankungen wie Rheuma und Arthritis verstärkt auf. Darüber hinaus konnte ihre Wirkung bei der Tumorbildung und Metastasierung verschiedener Tumore gezeigt werden. So ist beispielsweise die MMP 13 bei zahlreichen Malignomen hochreguliert.

Neben der Behandlung der zuvor genannten MMP-vermittelten Erkrankungen wie Rheuma und Arthritis, die bei einer Vielzahl älterer und zunehmend auch jüngerer Patienten auftreten, stellt auch die Heilung von Wunden, in denen naturgemäß erhöhte MMP 13-Konzentrationen nachgewiesen werden können, seit jeher ein Problem dar. Dieses gilt umsomehr für chronische Wunden, wobei unter chronischen Wunden solche verstanden werden, die nach 6 - 8 Wochen keine Tendenz zur Heilung zeigen und unter denen nach aktuellen Schätzungen ca. vier Millionen Menschen in Deutschland leiden, wobei jährliche Behandlungskosten in Höhe von zwei bis drei Milliarden Euro anfallen.

Ein wichtiger Faktor bei der Ausbildung dieser chronischen Wunden ist, wie bereits erwähnt, ein starkes Ungleichgewicht zwischen der Proteasekonzentration (MMP-Konzentration) im Wundsekret und der Konzentration der korrespondierenden Proteasehemmer (TIMP-Konzentration). Normalerweise erfolgt die Regulierung der MMPs, die nach der Sekretion zunächst aktiviert werden, durch die TIMPs (Tissue inhibitors of metalloproteinases). Bei einer Störung dieses empfindlichen Gleichgewichts liegt jedoch ein Überschuß an Proteasen vor, der nicht nur neu gebildetes, zur Wundheilung und zum Wundschluß erforderliches Gewebe sofort wieder abbaut, sondern auch gesundes Gewebe angreift und lysiert und Wachstumsfaktoren deaktiviert, wodurch es zu einer kontinuierlichen, teilweise schleichenden Verschlechterung der Wunde kommt.

Studien zur Wundheilung haben gezeigt, daß dagegen in MMP-13-defizienten Wunden die Wundheilung in starkem Maße gefördert und beschleunigt ist.

Eine stärkere Hemmung und eine geringere Aktivität bzw. Konzentration der MMPs, insbesondere der MMP 13, wäre somit ein geeigneter Ansatzpunkt für die Behandlung chronischer Wunden sowie arthritischer und rheumatischer Erkrankungen.

In *vitro* können MMPs durch Chelatbildner wie EDTA inhibiert werden, die das Zink des katalytischen Zentrums oder das strukturell relevante Kalium binden oder blockieren.
Darüber hinaus sind zahlreiche pharmakologische Präparate bekannt, die die Aktivität der MMPs hemmen. Allerdings sind diese Hemmer oft wenig spezifisch, so daß sie neben MMPs auch andere Enzyme beeinflussen oder sogar eine zytotoxische oder toxische Wirkung auf den gesamten Organismus ausüben.

Eine alternative, im Stand der Technik bekannte Behandlungsmethode für chronische Wunden ist beispielsweise die Applikation von kollagenhaltigen Wundauflagen, wodurch den in der Wunde sezernierten Proteasen ein alternatives Substrat angeboten wird und die abbauenden Enzyme kompetitiv gehemmt werden sollen.

Ein Problem dieser Behandlung ist, daß die Proteasen eine mangelnde Spezifität gegenüber den verwendeten Kollagenauflagen aufweisen und die Aktivität der MMPs durch den Kollagenüberschuß nur unzureichend gehemmt wird. Ein weiteres Problem ist, daß diese Kollagenauflagen meist aus Rindersehnen oder Häuten gewonnen werden. Bei dieser Kollagenquelle besteht die latente Gefahr und bei Verbrauchern immer noch die verbreitete Angst, daß die Wundauflage BSE-Erreger (BSE = Bovine Spongioforme Enzephalopathie) enthalten könnten. Mit der Auswahl alternativer Quellen wie z.B. Pferd oder Schwein kann diese Problematik zwar weitgehend umgangen werden, aber auch hier besteht das Restrisiko einer Infektion mit TSE-Erregern (TSE = Transforme Spongioforme Enzephalopathie).

Aufgabe der vorliegenden Erfindung ist daher, eine pharmazeutische Zubereitung oder ein pharmazeutisches Hilfsmittel zur Verfügung zu stellen, das die Aktivität der MMPs hemmt und die Heilung chronischer Wunden verbessert, ohne dabei die inhärente Gefahr einer Infektion mit Erregern einer spongioformen Enzephalopathie zu tragen, toxische Wirkungen zu zeigen oder andere unerwünschte Arzneimittelwirkungen aufzuweisen.

Überraschenderweise hat sich gezeigt, daß pharmazeutische Zubereitungen, die aus marinen Schwämmen *(Porifera)* gewonnenes Kollagen ("marines Kollagen"), insbesondere aus *Chondrosia reniformis* gewonnenes Kollagen, enthalten, eine stark hemmende Wirkung auf die MMP-Aktivität und insbesondere auf MMP 13 ausüben, so daß pharmazeutische Präparate auf Basis dieses Kollagens zur Behandlung von chronischen Wunden, die nach 6 bis 8 Wochen keine Tendenz zur Heilung zeigen, eingesetzt werden können.

Demzufolge bezieht sich die vorliegende Erfindung auf die Verwendung von aus marinen Schwämmen *(Porifera)* gewonnenem Kollagen zur Herstellung einer die Aktivität von Matrix-Metall-Proteasen hemmenden pharmazeutischen Zubereitung zur Behandlung von chronischen Wunden, die nach 6 bis 8 Wochen keine Tendenz zur Heilung zeigen.

Ein Vergleich zu handelsüblichen Kollagenzubereitungen (z.B. aus bovinem oder porcinem Kollagen) zeigte keine Aktivitätsbeeinträchtigung der Proteasen gegenüber dem Kontrollexperiment. "Marines Kollagen" wird gemäß vorliegender Erfindung dabei als Kollagen definiert, das aus marinen Schwämmen (*Porifera*) gewonnen worden ist.

Ein weiterer Vorteil dieser Zubereitungen mit "marinem Kollagen" ist, daß der Quellorganismus (mariner Schwamm) kein Nervensystem besitzt, so daß die Gefahr einer Übertragung von TSE-Erregern und eine damit einhergehende Infektion völlig ausgeschlossen ist.

Weiterhin besitzen die erfindungsgemäß Zubereitungen zur Behandlung von chronischen Wunden (wie oben definiert) nur ein sehr geringes allergenes Potential und die Zubereitungen können vom Organismus vollständig abgebaut werden.

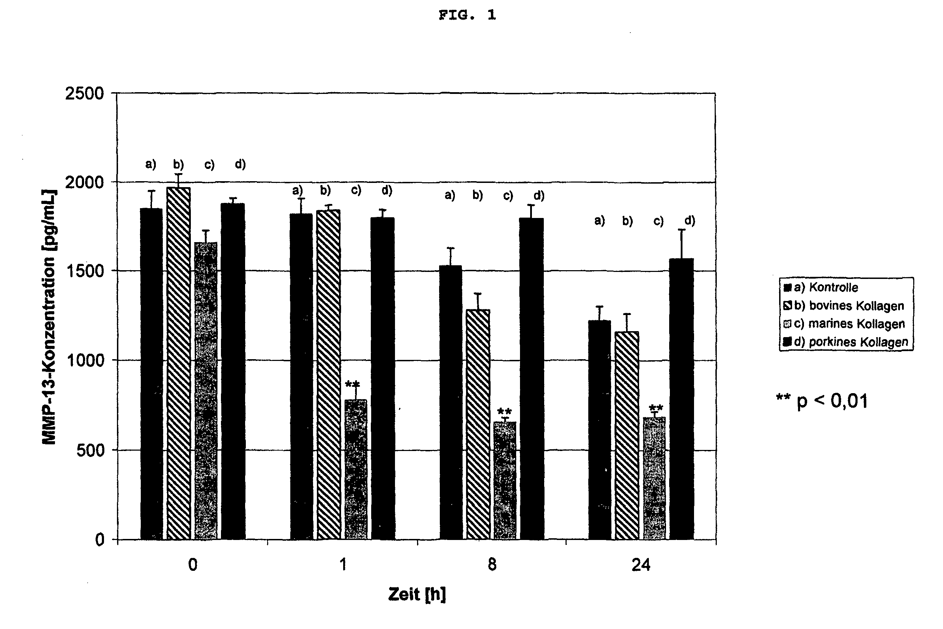

Wie bereits dargelegt, hemmen Zubereitungen mit aufbereitetem marinen Kollagen, das aus Schwämmen *(Porifera),* bevorzugt aus *Chondrosia reniformis,* gewonnen worden ist, die Aktivität von MMPs signifikant (s. Figur 1). Die Abnahme der Konzentration und somit der Aktivität der MMPs zeigt sich dabei bereits eine Stunde nach Applikation der Zubereitung. Die Zugabe weiterer Inhibitoren ist nicht erforderlich, da im Gegensatz zu den bekannten Präparaten die Kollagenpräparate der vorliegenden Erfindung MMPs hinreichend inhibieren und binden.

Das für die pharmazeutischen Zubereitungen der vorliegenden Erfindungen verwendete Kollagen wird durch Extraktion der Kollagenfraktion aus *Chondrosia reniformis* und anschließende Reinigung des Extraktes gewonnen, wobei eine Kollagenlösung erhalten wird.

In einer Ausführungsform liegt die erfindungsgemäße pharmazeutische Zubereitung zur Behandlung von chronischen Wunden als sterile Kollagenlösung vor. Die Lösung kann zur Behandlung von chronischen Wunden (wie oben definiert) verwendet werden.

Zur Behandlung von chronischen Wunden können beispielsweise handelsübliche, kollagenfreie Wundauflagen mit der oben erwähnten Kollagenlösung getränkt werden.

Eine weitere Ausführungsform sieht vor, daß das Kollagen aus der dargestellten Lösung durch Veränderung des pH-Wertes oder durch Zugabe von Ethanol oder anderer geeigneter, physiologisch akzeptabler Lösemittel zur Herstellung eines Kolloids oder einer Dispersion ausgefällt wird.

Die Viskosität des erhaltenen Kolloids oder der Dispersion kann durch Reduktion der Lösemittel, z.B. durch Entfernung im Vakuum oder durch Zentrifugation, oder durch Viskositätsmodifikatoren wie PVP (Polyvinylpyrrolidon), Polyacrylaten oder Cellulosederivaten, wie z.B. Carboxymethylcellulose (CMC), Hydroxyethylcellulose (HEC), Hydroxyethylpropylcellulose (HEPC) oder Methylcellulose (MC), eingestellt werden, so daß ein Gel oder eine hochviskose Lösung entsteht.

Sowohl die Dispersion oder das Kolloid als auch das Gel können dann zur Behandlung von chronischen Wunden (wie oben definiert) verwendet werden.

Eine weitere Ausführungsform sieht vor, daß das aus einem marinen Schwamm gewonnene, gereinigte Kollagen aus der Lösung ausgefällt, getrocknet und zu einem Granulat verarbeitet wird, das in Zubereitungen wie Cremes und Salben oder Wundauflagen eingearbeitet wird.

In einer bevorzugten Ausführungsform sind die erfindungsgemäßen Zubereitungen zur Behandlung von chronischen Wunden poröse Schwämme, die ein aus marinen Schwämmen *(Porifera)* gewonnenes Kollagen enthalten, bevorzugt Kollagen, das aus *Chondrosia reniformis* gewonnen und gemäß dem Verfahren nach Beispiel 1 aufbereitet wurde, wobei Wundauflagen zur Hemmung der MMPs, die den Wundschluß beschleunigen, besonders bevorzugt allein aus marinem Kollagen bestehen.

Die Kollagenzubereitungen zur Behandlung von chronischen Wunden können dabei neben anderen Wirk- und Hilfsstoffen zusätzlich mindestens einen weiteren, die MMPs nicht hemmenden Wirkstoff enthalten, beispielsweise nichtstercidale Antirheumatika wie Acetylsalicylsäure oder Ibuprofen und/oder Antibiotika.

Zur Herstellung der Wundauflagen wird eine aus *Chondrosia reniformis* extrahierte und gemäß der Vorschrift aus DE 10 2005 008 416 A1 (siehe Beispiel 1) aufgearbeitete Kollagenlösung gefriergetrocknet, wobei ein poröser Kollagenschwamm entsteht. Der so erhaltene Kollagenschwamm kann in flächige Stücke entsprechender Größe zerteilt und bis zur Verwendung steril verpackt gelagert werden. Um die Lagerfähigkeit des Produktes zu verbessern und um einer Infektion im Produkt verbliebene Erreger vorzubeugen, können der Kollagenlösung antimikrobielle Wirkstoffe zugesetzt, die verpackten Einheiten bestrahlt (Dosis: 25 kGy) und/oder mit Ethylenoxid begast werden.

Die Vorteile der so erhaltenen Wundauflagen sind, daß von ihnen keinerlei Infektionsgefahr mit TSE-Erregern ausgeht und die im Wundexsudat vorhandenen MMPs, insbesondere MMP 13, deaktiviert und die Wundheilung gefördert werden. Die Deaktivierung der MMPs ist dabei zum einen auf eine kompetitive Hemmung des Substrats zurückzuführen, da das in den Wundauflagen vorhandene, aus marinen Schwämmen gewonnene Kollagen entweder bevorzugt oder aber langsamer als das natürliche Substrat abgebaut wird, zum anderen darauf, daß in die Auflage aufgenommenes Exsudat gebunden und die MMP 13 immobilisiert wird, so daß ein "Rückfluß" zur Wundoberfläche und somit der Abbau neu gebildeter Strukturen verhindert und die Wundheilung verbessert wird.

Wie aus Figur 1 ersichtlich, ist eine deutliche Verminderung der Aktivität der MMP 13 bereits 60 min nach Zugabe des Kollagens zu beobachten, während bei handelsüblichen Kollagenzubereitungen eine deutlich höhere Aktivität der MMP 13 und keine Veränderung gegenüber dem Kontrollexperiment zu sehen ist. Die hier gezeigte schnelle und effiziente Aktivitätsminderung der MMP 13 ist aber, wie bereits oben beschrieben, eine der Grundvoraussetzungen, um die Wundheilung bei chronischen Wunden einzuleiten. Die Zugabe eines weiteren Inhibitors der MMP 13, z.B. eines unspezifischen Chelatbildners, ist bei den erfindungsgemäßen Zubereitungen zur Behandlung von chronischen Wunden nicht erforderlich.

Die Immobilisierung der MMPs in der Kollagenmatrix kann in einer weiteren Ausführungsform dadurch verstärkt werden, daß wundseitig eine semipermeable Membran auf dem Kollagenschwamm angeordnet ist, die die Aufnahme von Exsudat in den Schwamm gestattet, den Rückfluß von Exsudat und Abbauprodukten aber verhindert.

Ein weiterer Vorteil der so dargestellten Wundauflagen ist ihr aufgrund der hohen Reinheit geringes allergenes Potential und die vollständige biologische Abbaubarkeit auch in der Wunde.

Neben Kollagenschwämmen können mittels modifizierter Verfahren wie Ausstreichen und Trocknen auch Kollagenfilme hergestellt werden.

Darüber hinaus ist die Beschichtung von geeigneten Trägermaterialien wie Folien und Textilgewebe mit Kollagenfilmen und -schwämmen möglich, wobei die Folien und Gewebe sowohl eine undurchlässige als auch eine semipermeable Rückschicht bilden können.

Eine weitere Ausführungsform sieht vor, daß die Rückschicht zusätzlich auf eine der zuvor beschriebenen Auflagen aufgebracht wird, wobei bevorzugt die Fläche der Rückschicht gröβer als die Fläche des Kollagenschwammes ist und die überstehenden Bereiche mit einem hautverträglichen Haftkleber beschichtet sind, so daß die Wundauflage auf der Haut über der Wunde fixiert werden kann.

Die oben genannten pharmazeutischen Zubereitungen zur Behandlung von chronischen Wunden können weiterhin Konservierungsstoffe und antimikrobiell wirksame Verbindungen (z.B. Silbersulfadiazin, Biguanide, Polyhexanid, Nitroxolin, Octenidin, Taurolidin, Chlorhexidin, Benzalkonium-Halogenide und pharmakologisch akzeptable Salze oder Derivate der genannten Verbindungen), Viskositätsmodifikatoren (z.B. Polyvinylpyrrolidon oder Acrylate), Wachstumsfaktoren und andere Wundheilungsfaktoren, Hautschutzmittel (Fettsäuren, Fettsäureester) und dergleichen enthalten.

Darüber hinaus ist eine weitere Sterilisierung der Produkte mittels Strahlung (25 kGy), durch Begasung mit Ethylenoxid oder mit anderen geeigneten und dem Fachmann auf dem Gebiet bekannten Mitteln vorgesehen.

In den folgenden Beispielen werden bevorzugte Ausführungsformen der Erfindung dargestellt.

### Beispiel 1: Kollagenaufbereitung

Nach dem in der DE 10 2005 008 416 A1 beschriebenen Verfahren wurde ein in einem sauren Medium (pH 3) ausgefälltes Kollagenpräzipitat aus Chondrosia reniformis durch Filtration vom Medium abgetrennt und die Feuchtigkeit auf einen Restfeuchtigkeitsgehalt von etwa 84 Gew.-% reduziert. Anschließend wurden 121 Gramm der Kollagenrohmasse unter zweistündigem Rühren in 1300 ml einer wäßrigen 0,5 %-igen (v/v) H₂O₂-Lösung suspendiert und der pH der Lösung mittels einer 5 N NaOH-Lösung auf einen Wert von 12,4 eingestellt, um die Kollagenfasern in Lösung zu bringen. Die resultierende Kollagenlösung wurde zur Entfernung unlöslicher Bestandteile filtriert und anschließend unter kräftigem Rühren in 2600 ml Ethanol (Konz. 98 %) oder, abweichend von der DE 10 2005 008 416 A1, in eine HCl-Lösung mit einem pH von 0-3 gegeben und während der Zugabe in den Grenzen von 0-3 gehalten, wobei das Kollagen mit einer weißen oder leicht gelblichen Farbe in faseriger Form ausfiel. Die Kollagenfasern wurden durch Filtration vom Medium abgetrennt, von anhaftender Feuchte befreit und anschließend unter Rühren in 300 ml Reinstwasser homogen suspendiert. Der pH der Suspension wurde mit einer 5 N HCl-Lösung auf einen Wert von 6,5 eingestellt. Die so erhaltene Kollagenlösung hatte eine Kollagen-Konzentration von 2,8 Gew.-%.

Alle Verfahrensschritte wurden bei Raumtemperatur durchgeführt.

Um gemäß einer alternativen Ausführungsform eine sterile Kollagenlösung zu erhalten, wurden sämtliche Objekte, die mit dem Kollagen in Kontakt kommen, vor ihrer Verwendung mit einer 0,5 %-igen (v/v) H₂O₂-Lösung gespült und die gefällten Kollagenfasern nicht in Wasser, sondern in 300 ml einer wäßrigen 0,5 %-igen (v/v) H₂O₂-Lösung suspendiert.

### Beispiel 2:

Aus der in Beispiel 1 hergestellten Zubereitung wird unter Verwendung von Verdickern (z.B. PVP (Polyvinylpyrrolidon), Polyacrylaten oder anderen, dem Fachmann bekannten Creme- und Salbenbasen) eine Creme oder Salbe zur kutanen Anwendung hergestellt, die bei Applikation in einer Wunde die MMPs hemmt und den Wundschluß fördert.

Ein besonderer Vorteil dieser Zusammensetzung ist der noch in der Salbe verbliebene Wasserstoffperoxidanteil der Kollagenlösung, der zum einen die Haltbarkeit der Zubereitung erhöht und zum anderen in der Wunde eine antiseptische Wirkung entfaltet.

In einer anderen Ausführungsform kann die Kollagenlösung kurzzeitig erhitzt oder mit einem Reduktionsmittel oder Katalysator versetzt werden, um das Restperoxid zu zerstören.

### Beispiel 3:

Die nach Beispiel 1 hergestellte Kollagenlösung wird mit 0,5% Wasserstoffperoxidlösung oder mit Reinstwasser auf eine Kollagenkonzentration von 1-2% eingestellt. In der bevorzugten Ausführung wird die Kollagenkonzentration auf 1 Gew.-% und der pH Wert auf 6,1 eingestellt. Die Lösung wird in einer Schale ausgebracht und gefriergetrocknet. Der so erhaltene Kollagenschwamm kann als Wundauflage zur Behandlung schlecht heilender oder chronischer Wunden verwendet werden.

### Beispiel 4:

In einer weiteren Ausführungsform werden der Kollagenlösung vor der Gefriertrocknung antibiotische oder antimikrobielle Wirkstoffe zugesetzt.

Dazu wird der nach Beispiel 1 hergestellten Kollagenlösung unter starkem Rühren eine zuvor in 0,5 %-iger (v/v) H₂O₂-Lösung oder Reinstwasser gelöste antimikrobielle Substanz zugesetzt bis der Kollagengehalt 1 Gew.-% beträgt. Die Konzentration der antimikrobiellen Sustanz liegt zwischen 0,5 - 2 Gew.-% bezogen auf das Trockengewicht des Kollagens. Vorzugsweise ist die antimikrobielle Substanz Polyhexamethylenbiguanid-Hydrochlorid (Polymerisationsgrad = 12-18) mit einer Konzentration von 1 Gew.-% bezogen auf das Trockengewicht des Kollagens. Die so hergestellten antimikrobiellen Lösungen bzw. Dispersionen werden in eine Schale gegeben eingefroren und anschließend gefriergetrocknet.

### Beispiel 5:

In einem weiteren Ausführungsbeispiel wird eine antimikrobielle Wundauflage durch Imprägnierung mit einer antimikrobiellen Substanz hergestellt.

Dazu werden nach Beispiel 3 hergestellte Kollagenschwämme mittels im Stand der Technik bekannter Verfahren (z.B. Beschichten oder Besprühen) mit einer antimikrobiell wirksamen Substanz imprägniert. Hierbei wird eine konzentrierte wässrige oder alkoholische Lösung (10-50%) einer antimikrobiellen Substanz hergestellt und durch Sprühen oder Beschichten auf den Kollagenschwamm aufgebracht, so daß eine Konzentration der antimikrobiellen Substanz von 0,5 - 2 Gew.-% bezogen auf das Trockengewicht des Kollagens erhalten wird.

In einer bevorzugten Ausführung wird eine wäßrige oder eine ethanolische Polyhexamethylenbiguanid-Hydrochlorid-lösung (20 Gew.-%) durch Sprühen auf den Kollagenschwamm in einer Konzentration von 50 µl Lösung pro Gramm Kollagenschwamm aufgebracht, so daß eine Polyhexamethylenbiguanid-Hydrochlorid-Konzentration von 1 Gew.-% bezogen auf das Trockengewicht des Kollagens erhalten wird.

### Beispiel 6:

Die Versuche zur Bestimmung der Hemmung der MMP-13 durch die erfindungsgemäßen Kollagenzubereitungen wurden wie folgt durchgeführt.

### 1. Probenvorbereitung

Für die Experimente zur Hemmung der MMP-13 wurde aus lyophilisierten Proteinstandards eine Lösung mit einer Konzentrationen 2000 pg/mL hergestellt.

Aus den gemäß der vorliegenden Erfindung hergestellten Zubereitungen sowie aus handelsüblichen Kollagenauflagen wurden mit einer 8 mm Biopsie-Stanze (Stiefel Laboratorium GmbH, Offenbach, Germany) Stücke einheitlicher Größe (je 0,5 cm²) ausgestanzt und in 24-Well-Zellkulturplatten überführt. Jede Probe wurde in 1 mL Proteinlösung ausgenommen und anschließend für 24 h auf einem Schüttler (ThermoStar, BMG Labtech GmbH, Offenburg) bei 37°C inkubiert. Die Abnahme des Überstandes zur Kontrolle der MMP-13-Konzentration erfolgte nach 0, 1, 8 und 24 h, wobei die Proben sofort bei -20°C bis zur Messung eingefroren wurden. Als Kontrollexperiment wurden Proben ohne Kollagenzubereitung gemessen.

### 2. Bestimmung der MMP-13-Konzentration

Die Matrix-Metalloproteinase-13-Konzentration wurden mit Hilfe Enzym-markierter Immunosorbent-Assays (Quantikine pro-MMP-13 Immunoassay DM1300, R&D Systems GmbH, Wiesbaden) quantifiziert. Die Bestimmung der MMP-Konzentration erfolgte im Plattenreader (Fluostar, BMG Labtech GmbH, Offenburg) durch Messung der optischen Dichte (OD) bei 450 nm (Referenzwellenlänge: 620 nm). Anschließend konnte die Enzymkonzentration anhand einer "lin-log" Auftragung (OD bzw. Fluoreszenz - lineare Skala; Konzentration - Logarithmische Skala) mittels 4-Parameter-Fit berechnet werden. Alle Proben wurden in Doppelbestimmung gemessen, die erhaltenen Werte gemittelt und der Standardfehler berechnet.

Die MMP-13-Konzentration wurde für jeden Mess-Zeitpunkt durch zwei bis vier unabhängige Ansätze bestimmt. Die einzelnen Messungen erfolgten in Doppelbestimmung. Die in Figur 1 angegebenen Konzentrationen sind die gemittelten Werte aus jeweils 4, 6 bzw. 8 Messdaten unter Berücksichtigung des Standardfehlers. Zur Ermittlung der statistischen Signifikanz wurde eine einfache Varianzanalyse (ANOVA one way) durchgeführt.

## Patentansprüche

1. Verwendung von aus marinen Schwämmen (Porifera) gewonnenem Kollagen zur Herstellung einer die Aktivität von Matrix-Metallo-Proteasen hemmenden pharmazeutischen Zubereitung zur Behandlung von chronischen Wunden, die nach 6 bis 8 Wochen keine Tendenz zur Heilung zeigen.

2. Verwendung nach Anspruch 1, **dadurch gekennzeichnet, daß** das Kollagen aus *Chondrosia reniformis* gewonnen worden ist.

3. Verwendung nach Anspruch 1 oder 2, **dadurch gekennzeichnet, daß** die Matrix-Metallo-Protease 13 gehemmt wird.

4. Verwendung nach einem der vorangehenden Ansprüche, **dadurch gekennzeichnet, daß** die Zubereitung Konservierungsstoffe und/oder antimikrobiell wirksame Verbindungen enthält, die aus der Gruppe ausgewählt sind, die Silbersulfadiazin, Biguanide, Polyhexamethylenbiguanidin, Polyhexamethylenbiguanidin-Hydrochlorid, Nitroxolin, Octenidin, Taurolidin, Chlorhexidin und Benzalkonium-Halogenide und/oder pharmakologisch akzeptable Salze oder Derivate dieser Verbindungen umfaßt.

5. Verwendung nach einem der vorangehenden Ansprüche, **dadurch gekennzeichnet, daß** die Zubereitung mindestens einen weiteren, Matrix-Metalloproteasen nicht hemmenden Wirkstoff enthält, vorzugsweise ein Antibiotikum oder ein nichtsteroidales Antirheumatikum, insbesondere Acetylsalicylsäure oder Ibuprofen.

6. Verwendung nach einem der vorangehenden Ansprüche, **dadurch gekennzeichnet, daß** die Zubereitung als als poröser Schwamm hergestellt ist.

7. Verwendung nach einem der vorangehenden Ansprüche, **dadurch gekennzeichnet, daß** die Zubereitung eine Wundauflage ist.

8. Verwendung nach einem der vorangehenden Ansprüche, **dadurch gekennzeichnet, daß** die Zubereitung als Lösung oder Suspension vorliegt.

## Claims

1. Use of collagen obtained from marine sponges (Porifera) for the production of a pharmaceutical preparation inhibiting the activity of matrix metalloproteinases for treating chronic wounds that after 6 to 8 weeks show no tendency to heal.

2. The use according to claim 1, **characterized in that** the collagen is obtained from *Chondrosia reniformis.*

3. The use according to claim 1 or 2, **characterized in that** the matrix metalloproteinase 13 is inhibited.

4. The use according to any one of the preceding claims, **characterized in that** the preparation contains preservatives and/or antimicrobially active compounds selected from the group comprising silver sulfadiazine, biguanide, polyhexamethylene biguanidine, polyhexamethylene biguanidine hydrochloride, nitroxoline, octenidine, taurolidine, chlorhexidine and benzalkonium halogenide and/or pharmacologically acceptable salts or derivatives of these compounds.

5. The use according to any one of the preceding claims, **characterized in that** the preparation contains at least one additional active ingredient that does not inhibit matrix metalloproteinases, preferably an antibiotic or a non-steroidal antirheumatic agent, particularly acetylsalicylic acid or ibuprofen.

6. The use according to any one of the preceding claims, **characterized in that** the preparation is produced in the form of a porous sponge.

7. The use according to any one of the preceding claims, **characterized in that** the preparation is a wound dressing.

8. The use according to any one of the preceding claims, **characterized in that** the preparation is present as a solution or a suspension.

## Revendications

1. Utilisation de collagène obtenu à partir d'éponges marines (Porifera) pour la production d'une préparation pharmaceutique qui inhibe l'activité de métalloprotéases matricielles pour le traitement de lésions chroniques qui, après un laps de temps de 6 à 8 semaines, ne manifestent aucune tendance à la guérison.

2. Utilisation selon la revendication 1, **caractérisée en ce que** le collagène a été obtenu à partir de *Chondrosia reniformis.*

3. Utilisation selon la revendication 1 ou 2, **caractérisée en ce qu'**on inhibe la métalloprotéase matricielle 13.

4. Utilisation selon l'une quelconque des revendications précédentes, **caractérisée en ce que** la préparation contient des conservateurs et/ou des composés à activité antimicrobienne, qui sont choisis parmi le groupe qui comprend la sulfadiazine d'argent, des biguanides, la polyhexaméthylènebiguanidine, le chlorhydrate de polyhexaméthylènebiguanidine, la nitroxoline, l'octénidine, la taurolidine, la chlorhexidine et des halogénures de benzalkonium et/ou des sels ou des dérivés pharmacologiquement acceptables de ces composés.

5. Utilisation selon l'une quelconque des revendications précédentes, **caractérisée en ce que** la préparation contient au moins une substance active supplémentaire qui n'inhibe pas les métalloprotéases matricielles, de préférence un antibiotique ou un antirhumatismal non stéroïdien, en particulier l'acide acétylsalicylique ou l'ibuprofène.

6. Utilisation selon l'une quelconque des revendications précédentes, **caractérisée en ce que** la préparation est fabriquée sous la forme d'une éponge poreuse.

7. Utilisation selon l'une quelconque des revendications précédentes, **caractérisée en ce que** la préparation est un pansement.

8. Utilisation selon l'une quelconque des revendications précédentes, **caractérisée en ce que** la préparation est présente sous la forme d'une solution ou d'une suspension.
